Europäisches Patentamt

(19) European Patent Office

Office européen des brevets

(11) Numéro de publication: **0 254 794**

**A1**

(12) **DEMANDE DE BREVET EUROPEEN**

(21) Numéro de dépôt: 86870106.1

(22) Date de dépôt: **31.07.86**

(51) Int. Cl.⁴: **B01L 3/00** , G01N 33/53

(43) Date de publication de la demande:
**03.02.88 Bulletin 88/05**

(84) Etats contractants désignés:
**AT BE CH DE FR GB IT LI LU NL SE**

(71) Demandeur: **LABORATOIRES SANDERS-PROBEL, S.A.**
**47-51 rue Henri Wafelaerts Saint-Gilles**
**B-1060 Bruxelles(BE)**

(72) Inventeur: **Havaux, Jean-Claude**
**4, avenue des Renards**
**B-4040 Tilff(BE)**

(74) Mandataire: **Vigneron, Jean**
**Cabinet VIGNERON 30 avenue Eugène**
**Godaux**
**B-1150 Bruxelles(BE)**

(54) **Colonne de réaction.**

(57) Colonne de réaction (1) comprenant, ménagées dans un élément tubulaire étanche (2) , des cellules superposées ( 3, 3'... 3ⁿ) séparées l'une de l'autre par une membrane filtrante (4) perméable aux liquides et aux gaz, la cellule d'extrémité (3) recevant l'échantillon à analyser et comprenant des moyens (5) pour y permettre l'introduction de l'échantillon et des moyens (6) agencés pour y créer une pression supérieure à la pression régnant dans les cellules (3'... 3ⁿ) , les membranes filtrantes (4) étant perméables aux liquides lorsqu'il existe une différence de pression entre les cellules, la cellule d'extrémité (3ⁿ) étant obturée par une membrane (4') qui est au moins perméable aux gaz.

FIG.1.

## COLONNE DE REACTION

La présente invention a pour objet une colonne de réaction pour l'analyse d'un échantillon liquide ou mis en dilution.

L'invention a pour but de procurer une colonne de réaction simple pouvant être diffusée, notamment, dans le grand public pour permettre d'effectuer aisément et sûrement des analyses bien déterminées.

A cet effet, suivant l'invention, cette colonne comprend, ménagées dans un élément tubulaire étanche, des cellules superposées, coaxiales audit élément et séparées l'une de l'autre par une membrane filtrante perméable aux liquides et aux gaz, une des cellules d'extrémité de la colonne étant destinée à recevoir l'échantillon à analyser, cette cellule comprenant des moyens pour y permettre l'introduction de l'échantillon et des moyens agencés pour y créer une pression supérieure à la pression régnant dans les autres cellules, les membranes filtrantes précitées étant agencées pour être perméables aux liquides lorsqu'il existe une différence de pression entre les cellules, l'autre cellule d'extrémité de la colonne étant obturée, à l'extrémité correspondante de l'élément tubulaire, par une membrane qui est au moins perméable aux gaz.

Dans le but de faciliter les analyses et d'éviter toute erreur dans les résultats de celles-ci, la cellule de la colonne suivant l'invention, destinée à recevoir l'échantillon à analyser comprend avantageusement des moyens permettant d'assurer la dilution dudit échantillon, tandis que les cellules associées à la cellule destinée à l'échantillon comprennent chacune les moyens nécessaires aux diverses phases de l'analyse, une phase de celle-ci s'effectuant dans chacune des cellules, la membrane susdite obturant la cellule d'extrémité, dans laquelle s'effectue la dernière phase de l'analyse et qui est opposée à la cellule recevant l'échantillon, étant uniquement perméable aux gaz.

Suivant une forme de réalisation particulièrement avantageuse de l'invention, la colonne de réaction est associée à un dispositif de prélèvement d'un échantillon d'un volume déterminé.

D'autres détails et particularités de l'invention ressortiront de la description des dessins annexés au présent mémoire et qui représentent, à titre d'exemples non limitatifs, des formes de réalisations particulières de la colonne de réaction suivant l'invention.

La figure 1 est une vue schématique, en élévation et en coupe, d'une colonne de réaction pour la détection d'anticorps Ig G anti-toxoplasmose.

La figure 2 est une vue partielle analogue à la figure 1, à plus grande échelle que celle-ci et illustrant une variante de la colonne représentée à ladite figure 1.

La figure 3 est une vue partielle analogue à la figure 1, illustrant une autre variante de la colonne représentée à ladite figure 1.

La figure 4 est une vue explosée en élévation, avec brisures partielles, d'une colonne de réaction suivant l'invention associée à un dispositif de prélèvement d'échantillon.

La figure 5 est une vue partielle analogue à la figure 4 et illustre une variante de la colonne représentée à ladite figure 3.

La figure 6 est une vue analogue à la figure 1 et montre la colonne de réaction illustrée à ladite figure 1 associée à une colonne de référence dépourvue d'échantillon à analyser et à travers de laquelle ne passe que le produit de dilution.

Dans les différentes figures, les mêmes notations de référence désignent des éléments identiques ou analogues.

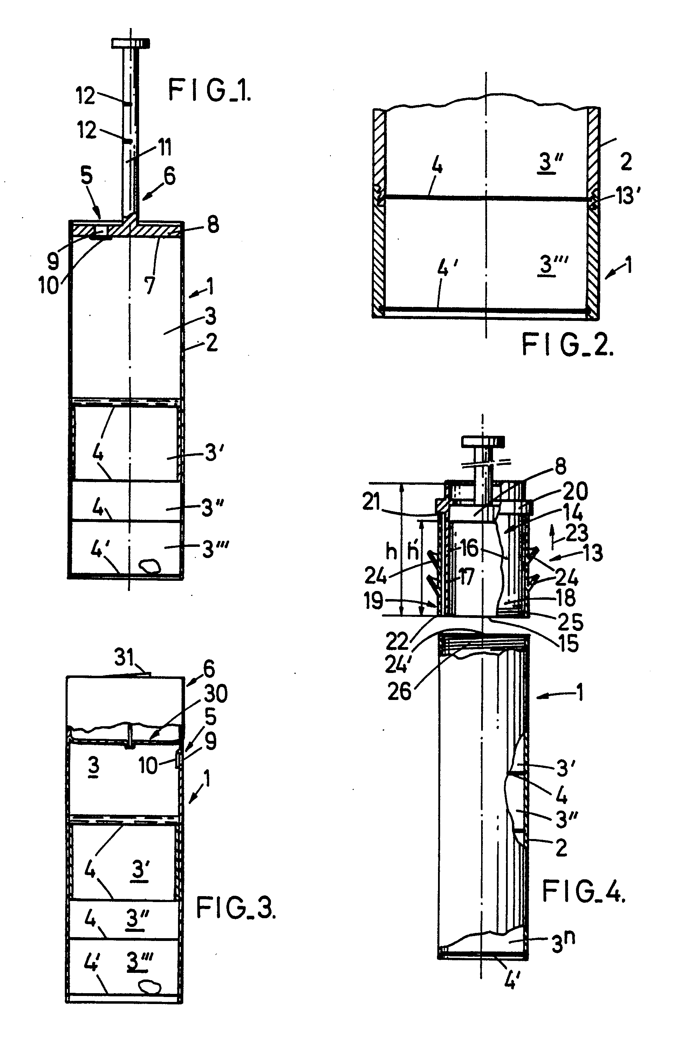

La colonne de réaction 1 suivant l'invention et illustrée aux figures 1, 2 et 4 à 6 est destinée à l'analyse d'un échantillon mis en dilution. Cette colonne comprend, ménagées dans un élément tubulaire 2 étanche et par exemple de section cylindrique, des cellules superposées $3, 3', 3'' ... 3^n$ qui sont coaxiales à l'élément 2 et qui sont séparées l'une de l'autre par une membrane filtrante 4 perméable aux liquides et aux gaz. La cellule 3, située à une des extrémités de la colonne, est destinée à recevoir l'échantillon à analyser et a un volume interne libre qui est au moins égal au volume interne total des cellules $3', 3'' ... 3^n$.

Cette cellule comprend des moyens 5 agencés pour y introduire l'échantillon, des moyens 6 agencés pour faire varier son volume interne afin de créer dans cette cellule 3 une pression supérieure à la pression régnant dans les cellules $3' ... 3^n$.

Les membranes filtrantes 4 normalement perméables aux gaz ne sont perméables aux liquides que lorsqu'il existe une différence de pression entre les cellules séparées par ces membranes. La cellule $3^n$, située à l'autre extrémité de la colonne, est obturée par une membrane 4' qui est au moins perméable aux gaz.

Pour faciliter l'analyse, la cellule 3 comprend avantageusement des moyens permettant d'assurer la dilution de l'échantillon et les cellules $3' ... 3^n$ comprennent avantageusement chacune les moyens nécessaires aux diverses phases de l'analyse, compte tenu du fait qu'une phase de cette analyse s'effectue dans chacune des cellules 3' ... $3^n$.

Dans le cas où la dernière phase de l'analyse s'effectue dans la cellule $3^n$ de la colonne, la membrane 4' obturant cette cellule $3^n$ est perméable aux gaz et imperméable aux liquides. dans le cas où la dernière phase de l'analyse s'effectue à l'extérieur de la colonne, par exemple pour que le résultat puisse être interprété par un professionnel des analyses, la cellule $3^n$, dans laquelle s'effectue l'avant dernière phase de l'analyse, a sa membrane 4' perméable aux gaz et aux liquides, la colonne se raccordant alors, au niveau de la cellule $3^n$, sur un récipient contenant les moyens nécessaires à cette dernière phase de l'analyse.

La colonne de réaction I,suivant l'invention et illustrée notamment aux figures 1 et 2, est destinée à la détection d'anticorps Ig G anti-toxoplasmose par ELISA compétitif et comprend quatre cellules 3, 3', 3" et 3".

La cellule 3 est destinée à recevoir un échantillon de sang humain et est délimitée, dans l'élément tubulaire 2, par la face 7 d'un piston 8, agencé dans ledit élément 2 et constituant les moyens 6 précités, et la membrane 4 la séparant de la cellule 3'. Le piston 8 comprend une ouverture 9, fermée normalement par un clapet à ressort 10, à travers laquelle est introduit l'échantillon. Le volume libre maximum compris entre la face 7 du piston 8 et ladite membrane 4 est au moins égal à la somme des volumes libres des cellules 3', 3" et 3".

Cette cellule 3 comprend, pour $25\mu l$ de sang, le milieu de dilution de 1,25 ml.

Les parois internes de l'élément tubulaire 2 comprises dans la cellule 3' sont revêtues d'antigènes de toxoplasmose ( $4\mu g/ml$) liées avec des anticorps humains Ig G anti-toxoplasmose conjugués à de la phosphatase alcaline (4 $\mu g/ml$).

La cellule 3" contient ± $1\mu g$ de paranitrophényl phosphate, substrat de la phosphatase alcaline.La cellule 3" contient un morceau de Na OH destiné à inhiber l'activité enzymatique. L'élément tubulaire 2 est, au moins au niveau de la cellule 3", transparent afin de pouvoir observer la coloration du liquide aboutissant dans cette cellule 3". La tige 11 du piston 8 est pourvue de repères 12 indiquant le degré d'enfoncement du piston 8 dans la cellule 3 pour chaque phase successive de l'analyse.

Cette colonne fonctionne de la manière suivante : après dilution de l'échantillon de sang dans la cellule 3, une première pression est exercée sur la tige 11 du piston 8 pour filtrer le sang dilué à travers la membrane 4 et transférer le filtrat dans la cellule 3'. Les antigènes de toxoplasmose revêtant les parois de la cellule 3' se lient aux anticorps humains Ig G anti-toxoplasmose conjugués à la phosphatase alcaline et ces derniers entrent en compétition avec les Ig G contenus dans le filtrat précité et ce pendant un temps d'incubation de l'ordre de deux heures et à une température de l'ordre de 37°C. Une deuxième pression sur la tige du piston 11 permet alors de faire passer le liquide à travers la membrane 4 séparant la cellule 3' de la cellule 3", de ladite cellule 3' à la cellule 3". Dans cette cellule 3", qui contient la paranitrophényl phosphate, les Ig G marqués sont présents dans le liquide si l'échantillon de sang est positif, ces Ig G marqués trouvant dans cette cellule 3" leur substrat, ce qui pro voque une coloration du liquide. On laisse l'activité enzymatique se poursuivre dans cette cellule 3" pendant une période de l'ordre de trente minutes à 37°C. On exerce enfin une dernière pression sur la tige 11 du piston 8 pour transférer le liquide de la cellule 3" dans la cellule 3" où l'activité enzymatique est inhibée par le Na OH contenu dans cette dernière cellule. Les résultats d'analyse obtenus avec la colonne suivant l'invention sont largement suffisants pour apprécier la présence d'anticorps.

Comme montré à la figure 2, la cellule 3" est avantageusement séparable de la colonne, grâce à un pas de vis 13', pour permettre l'utilisation de cette cellule 3" pour soit la soumettre à un professionnel de l'analyse, soit la conserver.

Comme illustré à la figure 3, les moyens 6 agencés pour créer dans la cellule 3 une pression supérieure à la pression régnant dans les cellules 3' à 3", peuvent être constitués par une réserve de gaz sous pression 30 associée à ladite cellule 3 et communiquant avec cette dernière, un organe de commande 31 étant prévu sur cette réserve 30 pour libérer le gaz et le doser dans cette cellule 3. Les moyens 5 pour introduire l'échantillon dans la cellule 3 sont alors ménagés dans l'élément tubulaire 2. Dans ce cas, il n'est plus nécessaire que le volume interne de la cellule 3 soit au moins égal au volume interne total des cellules 3' à 3". Les moyens de dilution de l'échantillon peuvent avantageusement être compris dans le gaz sous pression.

Comme illustré à la figure 4, la colonne de réaction suivant l'invention est avantageusement associée, notamment pour l'analyse de selles, à un dispositif de prélèvement 13 qui sera décrit ci-après et qui offre le grand avantage , lorsqu'il est extrait de la matière après prélèvement, de présenter toutes ses parties externes totalement exemptes de selles, d'où facilité et agrément de l'emploi, propreté d'emploi et prélévement exact du volume déterminé.

Ce dispositif 13 comprend une enceinte 14 ouverte à sa base 15 par laquelle elle est fixée à la colonne 1 pour que sa partie interne communique avec la cellule 3 de ladite colonne, dans laquelle l'échantillon prélevé sera transféré à l'aide d'un piston 8 logé dans l'enceinte 14 et destiné à coopérer avec les parois internes de l'enceinte et celles de la cellule 3, les parois latérales 16 de l'enceinte 14 étant pleines, le volume libre de cette enceinte correspondant au volume de l'échantillon à prélever tandis que le volume de la cellule 3 est au moins égal, comme décrit ci-dessus, au volume total des autres cellules de la colonne d'analyse.

Les sections interne et externe de l'enceinte sont constantes et ses faces internes 17 et externes 18 sont lisses et le dispositif 13 comprend un fourreau 19 enserrant lesdites parois externes 18 à partir de la base 15 de celle-ci et sur une hauteur h au moins égale à la hauteur interne h' de l'enceinte 14, afin que ce fourreau 19 puisse coulisser librement sur ces parois externes 18. Une saillie externe 20 est agencée sur ces parois 18 pour prendre appui sur le bord 21 du fourreau 19, qui est opposé au bord 22 de ce dernier et qui est situé au niveau de la base 15 de l'enceinte 14 afin d'empêcher, le coulissement dudit fourreau sur l'enceinte, suivant la flèche 23, lorsque la base 5 de cette enceinte sera introduite dans les selles pour prélever l'échantillon, tandis que desaspérités 24, régulièrement réparties à l'extérieur du fourreau 19, sont agencées de manière à coopérer avec les selles pour maintenir le fourreau dans les selles quand l'enceinte 14 est extraite de celles-ci, le fourreau 19 coulissant alors librement sur ladite enceinte 14. Il ressort clairement de ce qui précède que les parois externes 18 du fourreau sont protégées des selles par le fourreau 19 lors de l'introduction du dispositif dans celles-ci ainsi que lors du retrait dudit dispositif, le fourreau étant maintenu dans les selles par les aspérités 24 et coulissant alors librement sur le fourreau.

Lorsque le dispositif 13 est associé à la colonne 1, après enlèvement de l'opercule 24' prévu sur celle dernière, grâce par exemple à des filets 25 et 26 prévus respectivement sur le dispositif et sur la colonne, le piston 8 est manoeuvré, comme décrit ci-avant, pour procéder aux différentes phases de l'analyse.

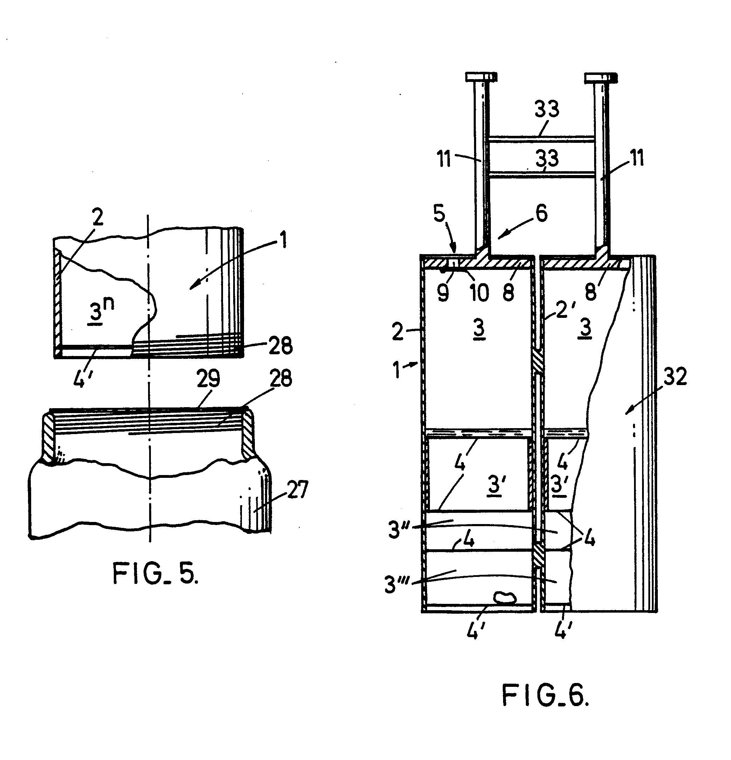

La figure 5 montre une colonne d'analyse 1 qui ne comprend pas de cellule destinée à la dernière phase de l'analyse, cette cellule étant constituée par un récipient séparé 27 qui sera par exemple utilisé par un professionnel de l'analyse pour apprécier le résultat final de celle-ci.

La colonne 1 et le récipient séparé 27 présentent des filets 28 qui permettent leur assemblage, après enlèvement de l'opercule 29 obturant le récipient 27.

Pour permettre une meilleure visualisation des résultats de l'analyse, la colonne de réaction 1 est avantageusement associée, comme montré à la figure 6, à une colonne de référence 32 dont l'élément tubulaire 2' est fixé à l'élément tubulaire 2 de la colonne 1. Cette dernière et la colonne 32 sont identiques à l'exception près que la colonne 32 ne comporte pas de moyens 5 pour l'introduction de l'échantillon dans sa cellule 3. En effet, seuls les moyens de dilution traversent cette colonne de référence. Les tiges 11 des pistons 8 des deux colonnes 1 et 32 sont réunies entre elles par des éléments de jonction 33 afin de permettre la commande simultanée des deux pistons et leur faire parcourir la même distance.

Ces éléments 33 jouent avantageusement le rôle des repères 12 précités indiquant le degré d'enfoncement des pistons dans les cellules 3 des colonnes 1 et 32 et se brisent un à un au fur et à mesure de l'enfoncement desdits pistons 8 dans les cellules 3.

Il doit être entendu que l'invention n'est nullement limitée aux formes de réalisation décrites et que bien des modifications peuvent être apportées à ces dernières sans sortir du cadre du présent brevet.

C'est ainsi qu'on pourrait notamment prévoir, plutôt que de ménager les cellules $3,...3^n$ dans l'élément tubulaire 2 , une colonne qui serait composée par un assemblage de cellules séparées, éventuellement interchangeables en fonction des analyses à effectuer.

**Revendications**

1. Colonne de réaction (1) pour l'analyse d'un échantillon, caractérisée en ce qu'elle comprend, ménagées dans un élément tubulaire (2) étanche, des cellules superposées $(3,3',3''...3^n)$, coaxiales audit élément (2) et séparées l'une de l'autre par une membrane filtrante (4) perméable aux liquides et aux gaz, une des cellules d'extrémité (3) de la colonne étant destinée à recevoir l'échantillon à analyser, cette cellule (3) comprenant des moyens (5) pour y permettre l'introduction de l'échantillon et des moyens (6) agencés pour y créer une pression supérieure à la pression régnant dans les autres cellules $(3'... 3^n)$, les membranes filtrantes (4) précitées étant agencées pour être perméables aux liquides lorsqu'il existe une différence de pression entre les cellules, l'autre cellule d'extrémité - $(3^n)$ de la colonne étant obturée, à l'extrémité correspondante de l'élément tubulaire (2) , par une membrane (4') qui est au moins perméable aux gaz.

2. Colonne de réaction suivant la revendication 1, caractérisée en ce que les moyens (6) précités, agencés pour créer dans la cellule (3) destinée à recevoir l'échantillon une pression supérieure à celle des autres cellules (3' ... 3$^n$) , sont des moyens, tels que piston, disposés dans cette cellule (3) pour faire varier son volume interne, le volume de cette cellule (3) destinée à recevoir l'échantillon étant au moins égal au volume total des autres cellules (3'... 3$^n$) de la colonne.

3. Colonne de réaction suivant la revendication 1, caractérisée en ce que les moyens (6) précités, agencés pour créer dans la cellule (3) destinée à recevoir l'échantillon une pression supérieure à celle des autres cellules, sont constitués par un gaz sous pression contenu dans un récipient (30) associé ou pouvant être associé à la cellule (3) destinée à recevoir l'échantillon.

4. Colonne de réaction suivant la revendication 3, caractérisée en ce que le gaz sous pression comprend les moyens de dilution de l'échantillon.

5. Colonne de réaction suivant l'une quelconque des revendications 1 à 3 , caractérisée en ce que la cellule (3) destinée à recevoir l'échantillon à analyser comprend des moyens permettant d'assurer la dilution de cet échantillon.

6. Colonne de réaction suivant l'une quelconque des revendications 1 à 5 , caractérisée en ce que les cellules (3' ...3$^n$) associées à la cellule (3)destinée à l'échantillon comprennent chacune les moyens nécessaires aux diverses phases de l'analyse, une phase de celle-ci s'effectuant dans chacune des cellules, la membrane (4') susdite obturant la cellule d'extrémité (3$^n$) , dans laquelle s'effectue la dernière phase de l'analyse et qui est opposée à la cellule (3) recevant l'échantillon, étant uniquement perméable aux gaz.

7. Colonne de réaction suivant la revendication 6, caractérisée en ce qu'au moins la zone de l'élément tubulaire précité correspondant à la cellule d'extrémité (3$^n$) dans laquelle s'effectue la dernière phase de l'analyse est perméable à la lumière, au moins dans les longueurs d'onde utilisées pour la mesure du résultat de l'analyse.

8. Colonne de réaction suivant l'une quelconque des revendications 1 à 5 , caractérisée en ce que les cellules (3'... 3$^n$) associées à la cellule (3) destinée à l'échantillon comprennent chacune les moyens nécessaires aux diverses phases de l'analyse à l'exception de la dernière, une phase de celle-ci s'effectuant dans chacune des cellules, la membrane (4') susdite obturant la cellule d'extrémité (3$^n$) dans laquelle s'effectue l'avant-dernière phase de l'analyse et qui est opposée à la cellule (3) recevant l'échantillon, étant perméable aux gaz et aux liquides.

9. Colonne de réaction suivant la revendication 8, caractérisée en ce qu'elle comprend, au niveau de la membrane (4') susdite obturant la cellule d'extrémité (3$^n$) dans laquelle s'effectue l'avant-dernière phase de l'analyse, des moyens (28) agencés pour permettre la fixation de l'élement tubulaire (2) sur un récipient (27) dans lequel s'effectuera la dernière phase de l'analyse, ce récipient (27) étant avantageusement perméable à la lumière, au moins aux longueurs d'onde précitées.

10. Colonne de réaction suivant l'une ou l'autre des revendications 8 et 9 , caractérisée en ce que la section de l'élément tubulaire (2) correspondant à la cellule d'extrémité (3''') dans laquelle s'effectue soit la dernière phase de l'analyse, soit l'avant-dernière phase de cette dernière, est détachable dudit élément tubulaire, des moyens étant prévus pour obturer cette section détachable, à l'opposé de la membrane perméable (4') précitée.

11. Colonne de réaction suivant l'une quelconque des revendications 6, 7 et 10, caractérisée en ce qu'elle comprend, pour la détection d'anticorps Ig G anti-toxoplasmose, quatre cellules ( 3 à 3''' ), une première cellule (3) destinée à recevoir un échantillon contenant du sang humain et pourvue du milieu de dilution, une deuxième cellule (3') dont les parois internes de l'élément tubulaire sont revêtues d'antigènes de toxoplasmose liées à des anticorps humains Ig G anti-toxoplasmose conjugués à de la phosphatase alcaline , une troisième cellule (3'') contenant du paranitrophényl phosphate, substrat de la phosphatase alcaline et une quatrième cellule (3''') contenant du NaOH capable d'inhiber l'activité enzymatique.

12. Colonne de réaction suivant l'une quelconque des revendications 4 à 11, caractérisée en ce qu'elle est associée à une colonne de référence (32), cette dernière étant identique à la colonne de réaction et n'étant traversée que par les moyens de dilution de l'échantillon, les moyens agencés pour créer la pression supérieure précitée dans la colonne de réaction et dans la colonne de référence étant également associés pour pouvoir être commandés simultanément et créer des pressions identiques dans les deux colonnes.

13. Colonne de réaction suivant l'une quelconque des revendications 1 à 12, caractérisée en ce que la cellule (3) destinée à recevoir l'échantillon est agencée pour pouvoir être associée à un dispositif (13)pour le prélèvement , dans une masse de matière, d'un volume déterminé de cette matière, en particulier pour le prélèvement d'un échantillon de selles, comprenant une enceinte (14) ouverte à sa base (15) et destinée à être raccordée coaxialement à la cellule (3) susdite, les sections externe et interne de l'enceinte étant constantes, la section interne de l'enceinte (14) correspondant à celle de l'élément tubulaire (2), les faces internes (17) et externes (18) de l'enceinte (14) étant lisses et le volume interne libre de l'enceinte correspondant au volume de l'échantillon à prélever, ladite enceinte (14) comprenant un fourreau (19) enserrant les parrois externes (18) de l'enceinte, à partir de sa base (15) et sur une hauteur (h) au moins égale à sa hauteur interne (h'), de manière à pouvoir coulisser sur ces parois externes (18), des moyens (20) agencés sur ces dernières pour prendre appui sur le bord (11) du fourreau (19), opposé au bord (22) de ce dernier situé au niveau de la base (15) de l'enceinte, afin d'empêcher le coulissement du fourreau sur l'enceinte lorsque la base de cette dernière est introduite dans la matière et des moyens (24), régulièrement répartis à l'extrémité dudit fourreau, agencés pour qu'ils coopèrent avec la matière pour maintenir ce fourreau dans cette dernière lorsque l'enceinte précitée est extraite de cette matière, les parois de l'enceinte étant pleines et des moyens (8) étant prévus sur cette enceinte (14) pour, d'une part dégager l'échantillon prélevé de l'enceinte et, d'autre part, faire varier le volume de la cellule (3) de la colonne de réaction (1) dans laquelle est introduit l'échantillon pour y créer une pression supérieure à la pression régnant dans les autres cellules (3'... 3ⁿ) de la colonne.

14. Colonne de réaction suivant la revendication 13, caractérisée en ce que les moyens (8) précités agencés pour dégager l'échantillon du dispositif de prélèvement (13) et pour faire varier le volume de la cellule (3) susdite sont constitués par un piston logé dans l'enceinte (14) et destiné à coopérer avec les parois internes (17) de cette dernière et avec les parois internes de la cellule (3) de la colonne recevant l'échantillon, la tige du piston faisant saillie par rapport à la paroi de l'enceinte opposée à la base (15) de celle-ci et étant guidée par cette paroi.

0 254 794

FIG_1.

FIG_2.

FIG_3.

FIG_4.

FIG. 5.

FIG. 6.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 091 793 (WILSON PHARMACEUTICAL & CHEMICAL CORP.) * En entier * | 1-3,10 | B 01 L 3/00 G 01 N 33/53 |
| A | US-A-3 519 390 (R.K. DICKEY et al.) * En entier * | 1,2,9 | |
| A | DE-A-2 028 822 (H. KELLER) * Figures 1,4; page 3, ligne 1 - page 6, ligne 16 * | 1,3,8 | |
| A | FR-A-2 154 175 (N.V. PHILIPS' GLOEILAMPENFABRIEKEN) * En entier * | 1,12 | |
| A | GB-A-2 005 831 (GIST-BROCADES N.V.) * Abrégé; page 2, lignes 3-19; revendications 1,2 * | 1,11 | DOMAINES TECHNIQUES RECHERCHES (Int. Cl.4) G 01 N B 01 L |
| A | US-A-3 825 410 (K.D. BAGSHAWE) | | |

Le présent rapport de recherche a été établi pour toutes les revendications

| Lieu de la recherche | Date d'achèvement de la recherche | Examinateur |
|---|---|---|
| LA HAYE | 23-03-1987 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82